# EUROPEAN PATENT SPECIFICATION

(11) **EP 4 517 490 B1**
(45) Date of publication and mention of the grant of the patent: **10.12.2025**
(21) Application number: 24179013.8
(22) Date of filing: 30.05.2024
(51) Int. Cl.: G06F 3/01, A61B 5/00, G02B 27/01, G06V 40/16, G06T 5/50, H04N 23/56, H04N 23/84, H04N 25/10, G02B 27/00, G06V 10/143, G06V 10/147, G06V 10/10, G06V 40/18, G06T 13/40, G06T 7/00

(54) **GENERATING REALISTIC AVATARS FOR EXTENDED REALITY**
ERZEUGUNG REALISTISCHER AVATARE FÜR ERWEITERTE REALITÄT
GÉNÉRATION D'AVATARS RÉALISTES POUR UNE RÉALITÉ ÉTENDUE

(30) Priority: 30.08.2023 US 202318458708
(43) Date of publication of application: 05.03.2025
(73) Proprietor: Meta Platforms, Inc., Menlo Park, CA 94025 (US)
(72) Inventor: Robertson, John Enders, Menlo Park, 94025 (US)
(74) Representative: Murgitroyd & Company

(56) References cited:
- WO-A1-2022/066400
- US-A1- 2019 012 528
- ANSPACH JORDAN ET AL: "Sequential RGB color imaging with a millimeter-scale monochrome camera with a rolling shutter", vol. 62, no. 17, 1 June 2023 (2023-06-01), US, pages 4496, XP093217238, ISSN: 1559-128X, Retrieved from the Internet <URL:https://opg.optica.org/directpdfaccess/23c9d46b-4fc6-485f-a06f3d1f5894d413_531174/ao-62-17-4496.pdf?da=1&id=531174&seq=0&mobile=no> [retrieved on 20241022], DOI: 10.1364/AO.488272

## Description

### TECHNICAL FIELD

This disclosure relates generally to extended reality environments, and, more particularly, to generating realistic avatars for extended reality environments.

### BACKGROUND

Extended reality is a form of reality that has been adjusted in some manner before presentation to a user, which may include, for example, a virtual reality (VR), an augmented reality (AR), a mixed reality (MR), a hybrid reality, or some combination and/or derivatives thereof. Extended reality content may include completely generated content or generated content combined with captured content (e.g., real-world photographs). The extended reality content may include video, audio, haptic feedback, or some combination thereof, and any of which may be presented in a single channel or in multiple channels (such as stereo video that produces a three-dimensional effect to the viewer). Extended reality may be associated with applications, products, accessories, services, or some combination thereof, that are, for example, used to create content in extended reality and/or used in (e.g., perform activities in) extended reality. The extended reality system that provides the extended reality content may be implemented on various platforms, including a head-mounted display (HMD) connected to a host computer system, a standalone HMD, a mobile device or computing system, or any other hardware platform capable of providing extended reality content to one or more viewers. In some extended reality applications, a user may manually create an avatar, which may include a virtual representation of the user as the user navigates and experiences an extended reality environment. However, manually creating an avatar may be time-consuming and cumbersome for users, especially in instances in which the user is desiring to commence a work-related extended reality application or experience, or other similar time-sensitive application or experience. Sterling Labs LLC describes in WO 2022/066400 A1 sensor emulation, in particular providing images based on image sensor emulation. Wilson et al describe in US 2019/0012528 A1 a facial expression recognition system, a facial expression recognition method and a facial expression recognition program. Anspach et al describes in Applied Optics, vol. 62, no. 17, 1 June 2023, page 4496, sequential RGB color imaging with a millimeter-scale monochrome camera with a rolling shutter.

### SUMMARY OF CERTAIN EMBODIMENTS

The present invention is directed to techniques and apparatus for generating a realistic avatar of a user based on detected or calculated visible wavelengths obtained utilizing monochromatic cameras suited for eye-tracking or face-tracking in extended reality (XR) display devices.

**In** a first aspect of the invention, there is provided a method as defined in claim 1. In a second aspect of the invention, there is provided an electronic device as defined in claim 9. In a third aspect of the invention, there is provided a non-transitory computer-readable medium as defined in claim 14.

**In** certain embodiments, the first color component comprises a red color component; the second color component comprises a green color component; and the third color component comprises a blue color component.

In certain embodiments, the first frame includes only the red color component; the second frame includes only the green color component; and the third frame includes only the blue color component.

In certain embodiments, determining, for each of the plurality of images of the user, the visible wavelengths comprises determining a color for each of a plurality of characteristics of the user.

In certain embodiments, the plurality of characteristics of the user comprises one or more of an eye color of the user, a head hair color of the user, a skin complexion of the user, a facial hair color of the user, or a cheek complexion of the user.

In certain embodiments, generating the realistic avatar of the user comprises combining the plurality of images of the user based on the visible wavelength for each of the first color component, the second color component, and the third color component.

In certain embodiments, the method further comprises: in lieu of displaying the sequence of frames to the user, projecting, by a plurality of light-sources of the electronic device, a sequence of optical pulses onto the user, wherein the sequence of optical pulses comprises a first optical pulse including only the first color component, a second optical pulse including only the second color component, and a third optical pulse frame including only the third color component.

In certain embodiments, the plurality of light-sources comprises a plurality of light-emitting diodes (LEDs).

In certain embodiments, the capturing, by the one or more cameras, the plurality of images of the user further comprises: capturing a first image of the plurality of images of the user while displaying the first frame including the first color component; capturing a second image of the plurality of images of the user while displaying the second frame including the second color component; and capturing a third image of the plurality of images of the user while displaying the third frame including the third color component.

In certain embodiments, the method further comprises determining a heart rate of the user based on the visible wavelength for each of the first color component, the second color component, and the third color component.

In certain embodiments, the method further comprises determining a blood oxygen level of the user based on the visible wavelength for each of the first color component, the second color component, and the third color component.

In certain embodiments, the first color component comprises a red color component; the second color component comprises a green color component; and the third color component comprises a blue color component.

In certain embodiments, the first frame includes only the red color component; the second frame includes only the green color component; and the third frame includes only the blue color component.

In certain embodiments, wherein the instructions to determine, for each of the plurality of images of the user, the visible wavelengths further comprise instructions to determine a color for each of a plurality of characteristics of the user.

In certain embodiments, the plurality of characteristics of the user comprises one or more of an eye color of the user, a head hair color of the user, a skin complexion of the user, a facial hair color of the user, or a cheek complexion of the user.

In certain embodiments, the instructions to generate the realistic avatar of the user further comprise instructions to combine the plurality of images of the user based on the visible wavelength for each of the first color component, the second color component, and the third color component.

In certain embodiments, the instructions further comprise instructions to: in lieu of displaying the sequence of frames to the user, project, by a plurality of light-sources of the electronic device, a sequence of optical pulses onto the user, wherein the sequence of optical pulses comprises a first optical pulse including only the first color component, a second optical pulse including only the second color component, and a third optical pulse frame including only the third color component.

In certain embodiments, wherein the instructions to capture, by the one or more cameras, the plurality of images of the user further comprise instructions to: capture a first image of the plurality of images of the user while displaying the first frame including the first color component; capture a second image of the plurality of images of the user while displaying the second frame including the second color component; and capture a third image of the plurality of images of the user while displaying the third frame including the third color component.

In embodiments, the instructions may further comprise instructions to cause the electronic device of the second aspect to perform any steps of the method of the first aspect.

In certain embodiments, an electronic device may display, by at least one display of the electronic device, a sequence of frames to a user of the electronic device. In one embodiment, the sequence of frames may include a first frame including a first color component, a second frame including a second color component, and a third frame including a third color component. For example, in some embodiments, the first color component may include a red color component, the second color component may include a green color component, and the third color component may include a blue color component. In certain embodiments, the first frame may include only the red color component, the second frame may include only the green color component, and the third frame may include only the blue color component.

In certain embodiments, in lieu of displaying the sequence of frames to the user, the electronic device may project, by a number of light-sources of the electronic device, a sequence of optical pulses onto the user. For example, the sequence of optical pulses may include a first optical pulse including only the red color component, a second optical pulse including only the green color component, and a third optical pulse frame including only the blue color component. In one embodiment, the number of light-sources may include a number of light-emitting diodes (LEDs). In certain embodiments, while displaying the sequence of frames to the user, the electronic device may then capture, by one or more cameras of the electronic device, a number of images of the user. For example, in some embodiments, capturing, by the one or more cameras, the number of images of the user may include capturing a first image of the number of images of the user while displaying the first frame including the red color component, capturing a second image of the number of images of the user while displaying the second frame including the green color component, and capturing a third image of the number of images of the user while displaying the third frame including the blue color component.

**In** certain embodiments, the electronic device may then determine, for each of the number of images of the user, a visible wavelength for each of the first color component, the second color component, and the third color component. For example, the electronic device may determine a visible wavelength for the red color component with respect to the first image, the green color component with respect to the second image, and the blue color component with respect to the third image. In certain embodiments, determining, for each of the number of images of the user, the visible wavelengths may include determining a color for each of a number of characteristics of the user. For example, in some embodiments, the number of characteristics of the user may include one or more of an eye color of the user, a head hair color of the user, a skin complexion of the user, a facial hair color of the user, or a cheek complexion of the user.

**In** certain embodiments, the electronic device may then generate a realistic avatar of the user based on the visible wavelength for each of the red color component, the green color component, and the blue color component. For example, in some embodiments, generating the realistic avatar of the user may include combining the number of images of the user based on the visible wavelength for each of the red color component, the green color component, and the blue color component. In certain embodiments, the electronic device may also determine a heart rate of the user based on the visible wavelength for each of the red color component, the green color component, and the blue color component. In certain embodiments, the electronic device may also determine a blood oxygen level of the user based on the visible wavelength for each of the red color component, the green color component, and the blue color component.

The embodiments disclosed herein are only examples, and the scope of this disclosure is not limited to them. Certain embodiments may include all, some, or none of the components, elements, features, functions, operations, or steps of the embodiments disclosed above.

### BRIEF DESCRIPTION OF THE DRAWINGS

FIG. 1 illustrates a cross-section of an example extended reality (XR) display device.
FIG. 2 depicts an illustrative example of generating a realistic avatar of a user based on detected or calculated visible wavelengths obtained utilizing monochromatic cameras suited for eye-tracking or face-tracking in an XR display device.
FIG. 3 illustrates a timing diagram that shows the timing of an eye-tracking and face-tracking functionality operation, a display operation, and a camera operation.
FIG. 4 illustrates a flow diagram of a method for generating a realistic avatar of a user based on detected or calculated visible wavelengths obtained utilizing monochromatic cameras suited for eye-tracking or face-tracking in an XR display device.
FIG. 5 illustrates an example network environment associated with a social-networking system.
FIG. 6 illustrates an example computer system.

### DESCRIPTION OF EXAMPLE EMBODIMENTS

The present embodiments are directed to techniques for generating a realistic avatar of a user based on detected or calculated visible wavelengths obtained utilizing monochromatic cameras suited for eye-tracking or face-tracking in extended reality (XR) display devices. In certain embodiments, an electronic device may display, by at least one display of the electronic device, a sequence of frames to a user of the electronic device. In one embodiment, the sequence of frames may include a first frame including a first color component, a second frame including a second color component, and a third frame including a third color component. For example, in some embodiments, the first color component may include a red color component, the second color component may include a green color component, and the third color component may include a blue color component. In certain embodiments, the first frame may include only the red color component, the second frame may include only the green color component, and the third frame may include only the blue color component.

In certain embodiments, in lieu of displaying the sequence of frames to the user, the electronic device may project, by a number of light-sources of the electronic device, a sequence of optical pulses onto the user. For example, the sequence of optical pulses may include a first optical pulse including only the red color component, a second optical pulse including only the green color component, and a third optical pulse frame including only the blue color component. In one embodiment, the number of light-sources may include a number of light-emitting diodes (LEDs). In certain embodiments, while displaying the sequence of frames to the user, the electronic device may then capture, by one or more cameras of the electronic device, a number of images of the user. For example, in some embodiments, capturing, by the one or more cameras, the number of images of the user may include capturing a first image of the number of images of the user while displaying the first frame including the red color component, capturing a second image of the number of images of the user while displaying the second frame including the green color component, and capturing a third image of the number of images of the user while displaying the third frame including the blue color component.

**In** certain embodiments, the electronic device may then determine, for each of the number of images of the user, a visible wavelength for each of the first color component, the second color component, and the third color component. For example, the electronic device may determine a visible wavelength for the red color component with respect to the first image, the green color component with respect to the second image, and the blue color component with respect to the third image. In certain embodiments, determining, for each of the number of images of the user, the visible wavelengths may include determining a color for each of a number of characteristics of the user. For example, in some embodiments, the number of characteristics of the user may include one or more of an eye color of the user, a head hair color of the user, a skin complexion of the user, a facial hair color of the user, or a cheek complexion of the user.

In certain embodiments, the electronic device may then generate a realistic avatar of the user based on the visible wavelength for each of the red color component, the green color component, and the blue color component. For example, in some embodiments, generating the realistic avatar of the user may include combining the number of images of the user based on the visible wavelength for each of the red color component, the green color component, and the blue color component. In certain embodiments, the electronic device may also determine a heart rate of the user based on the visible wavelength for each of the red color component, the green color component, and the blue color component. In certain embodiments, the electronic device may also determine a blood oxygen level of the user based on the visible wavelength for each of the red color component, the green color component, and the blue color component.

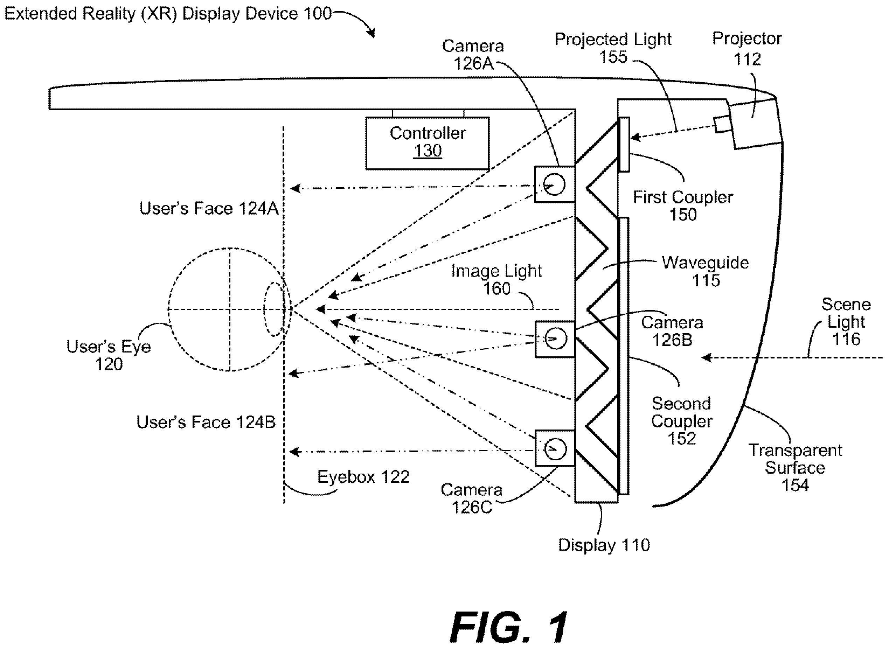

FIG. 1 illustrates a cross-section of an example extended reality (XR) display device 100, such as a head-mounted display (HMD) or XR glasses, in accordance with the presently disclosed embodiments. The XR display device 100 may include one or more displays 110, which may include at least one waveguide 115. It should be appreciated that the XR display device 100 as illustrated is an example of one embodiment of an HMD that may be useful in providing adaptive rending, in accordance with the presently disclosed embodiments. In another embodiment, the XR display device 100 may include a see-through HMD. which may not include a waveguide and may instead render images directly onto, for example, one or more transparent or semi-transparent mirrors that may be placed in front of the eyes of a user, for example. FIG. 1 also shows an eyebox 122, which is a location where a user's eye 120 may be positioned when the user wears XR display device 100. As long as a user' eye 120 is aligned with the eyebox 122, the user may be able to see a full-color image, or a pupil replication directed toward the eyebox 122 by the waveguide 115.

In certain embodiments, a location of where a user's face 124A, 124B may be positioned on either side of the user's eye 120 when the user wears the XR display device 100 may is also illustrated. The waveguide 115 may produce and direct many pupil replications to the eyebox 122. The waveguide 115 may be configured to direct image light 160 to the eyebox 122 located proximate to the eye 120. For purposes of illustration, FIG. 1 shows the cross-section associated with a single eye 120 and single waveguide 115. In certain embodiments, the waveguide 115 or another waveguide may provide image light to an eyebox located at another eye of the user. In certain embodiments, the waveguide 115 may be composed of one or more materials (e.g., plastic, glass, etc.) with one or more refractive indices that effectively minimize the weight and widen a field of view (FOV) of the display device 110.

In other embodiments, the display 110 may include one or more optical elements between the waveguide 115 and the user's eye 120. The optical elements may act to, for example, correct aberrations in the image light 160, magnify the image light 160, make some other optical adjustment of the image light 160, or perform a combination thereof. Examples of optical elements may include an aperture, a Fresnel lens, a refractive (e.g., convex and/or concave) lens, a reflective surface, a filter, or any other suitable optical element that affects image light. The waveguide 115 may include a waveguide with one or more sets of Bragg gratings, for example.

In one embodiment, a form of the display 110 that may be used in an XR display device 100 may be referred to as a scanline or one-dimensional ("1D") waveguide display. In this display, a row of a light source may generate the light that is used to illuminate the entire vertical space (or horizontal space, where appropriate) of the display. Multiple smaller images may be combined to form a larger composite image as perceived by the viewer. A scanning element may cause the source light, treated by waveguide components, to be output to the eye 120 of the user in a specific pattern corresponding to a generation pattern used by the emitters to optimize display draw rate. For example, the light source may first be provided color values corresponding to a single row of pixels along the top of a display image. The light may be transferred to the appropriate section of the eyebox 122 using a waveguide-based process assisted with a microelectromechanical system (MEMS)-powered oscillating mirror.

In certain embodiments, after a short period of time, the light source may be provided color values corresponding to the next row of pixels (e.g., below the first). The light for this section of the image may then use the same process to position the color values in the appropriate position. Scanning displays may utilize less power to run and may generate less heat than traditional displays comprised of the same emitters. Scanning displays may have less weight as well, owing in part to the quality of the materials used in the scanning element and optics system. The frame rate of the display may be limited based on the oscillation speed of the mirror.

In another embodiment, a form of the display 110 that may be used in an XR display device 100 may be a 2D or two-dimensional waveguide display. In such a display, no oscillating mirror may be utilized, as a light source may be used that includes vertical and horizontal components (e.g., in an array). Where the 1D variant lights the display on a row-by-row basis, the 2D variant may be capable of providing a significantly improved frame rate because the 2D variant may not be dependent on the oscillating mirror to provide for the vertical component of an image. To further improve the frame rate, the light source of a 2D waveguide display may be bonded to the controller and/or memory providing driving instructions for the display system. For example, the light source may be bonded to the memory that holds the color instructions for the display and/or the driver transistors. The result of such a configuration is that the light source for such a display may be operable with a considerably faster frame rate.

In certain embodiments, the XR display device 100 may include a light source such as a projector 112 that emits projected light 155 depicting one or more images. Many suitable display light source technologies are contemplated, including, but not limited to, liquid crystal display (LCD), liquid crystal on silicon (LCOS), light-emitting diode (LED), organic LED (OLED), micro-LED (µLED), digital micromirror device (DMD), or any combination thereof. The projected light 155 may be received by a first coupler 150 of the waveguide 115. The waveguide 115 may combine the projected light 155 with real-world scene light 116 received by a second coupler 152. The scene light 116 may be, for example, light from a real-world environment, and may pass through a transparent (or semi-transparent) surface 154 to the second coupler 152. In some embodiments the XR display device 100 may also include a number of light sources, such as a set of light-emitting diodes (LEDs), that may be utilized to project emitted light 155 onto the user's eye 120 and/or the user's face 124A, 124B.

In certain embodiments, the transparent surface 154 may be, for example, a protective curved glass or a lens formed from glass, plastic, or other transparent material. The coupling components of the waveguide 115 may direct the projected light 155 along a total internal reflection path of the waveguide 115. The scene light 116 may be seen by the user's eye 120. Furthermore, the projected light 155 may first pass through a small air gap between the projector 112 and the waveguide 115 before interacting with a coupling element incorporated into the waveguide, such as the first coupler 150. The light path, in some examples, may include grating structures or other types of light decoupling structures that decouple portions of the light from the total internal reflection path to direct multiple instances of an image, "pupil replications," out of the waveguide 115 at different places and toward the eyebox 122 of the XR display device 100.

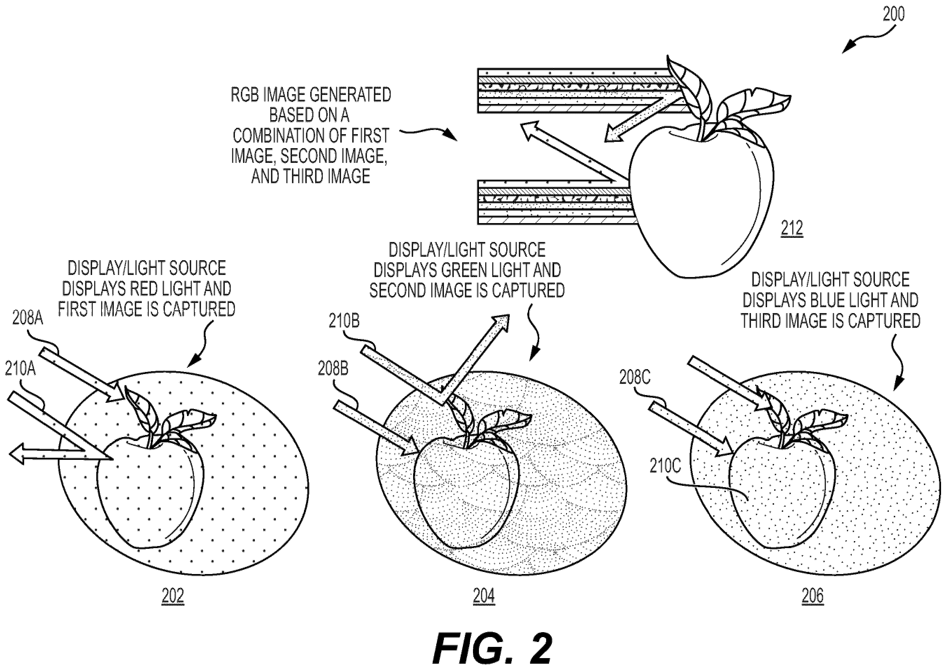

In certain embodiments, as further depicted by FIG. 1, the XR display device 100 may include a number of cameras 126A, 126B, and 126C. In certain embodiments, the number of cameras 126A, 126B, and 126B may include one or more infrared cameras 126A, one or monochromatic cameras 126B, and one or more depth cameras 126C that may be suitable for performing eye-tracking of the user's eye 120, face-tracking of the user's face 124A, 124B, or a combination of eye-tracking of the user's eye 120 and face-tracking of the user's face 124A, 124B. For example, in one embodiment, the one or monochromatic cameras 126B may operate in a narrow infrared band (e.g., 850 nanometers (nm)) and excluding visual spectrum data capture. In certain embodiments, as will be further appreciated below with respect to FIGS. 2-4, the one or monochromatic cameras 126B (or one or more other cameras of the number of cameras 126A, 126B, and 126B) may be utilized to for generate a realistic avatar of the user (e.g., including an eye color of the user, a head hair color of the user, a skin complexion of the user, a facial hair color of the user, a cheek complexion of the user, and so forth) by temporarily removing an infrared bandpass filter of the one or monochromatic cameras 126B and without receiving any inputs from the user.

In certain embodiments, one or more controllers 130 may control the operations of the projector 112 and the number of cameras 126A, 126B, and 126B. The controller 130 may generate display instructions for a display system of the projector 112 or image capture instructions for the number of cameras 126A, 126B, and 126B. The display instructions may include instructions to project or emit one or more images, and the image capture instructions may include instructions to capture one or more images in a successive sequence, for example. In certain embodiments, the display instructions and image capture instructions may include frame image color or monochromatic data. The display instructions and image capture instructions may be received from, for example, one or more processing devices included in the XR display device 100 of FIG. 1 or in wireless or wired communication therewith. The display instructions may further include instructions for moving the projector 112, for moving the waveguide 115 by activating an actuation system, or for moving or adjusting the lens of one or more of the number of cameras 126A, 126B, and 126B. The controller 130 may include a combination of hardware, software, and/or firmware not explicitly shown herein so as not to obscure other aspects of the disclosure.

**FIG.** 2 depicts an illustrative example 200 of the present techniques for generating a realistic avatar of a user utilizing monochromatic cameras suited for eye-tracking or face-tracking in XR display devices, in accordance with the presently disclosed embodiments. It should be appreciated that while the illustrative example 200 may be discussed theoretically herein with respect to a red apple in examples 202, 204, and 206, in the present embodiments, red light , green light, and blue light may be impinged onto a user's eye 120 and a user's face 124A, 124B. In certain embodiments, the display 110 of the XR display device 100 may display a sequence of frames (e.g., illustrated by red light 208A, green light 208B, and blue light 208C) to the user when the user first places the XR display device 100 onto her head. In one embodiment, the sequence of frames may include a first frame including only a red color component, a second frame including only a green color component, and a third frame including only a blue color component.

For example, as depicted by the illustrative example 200, the display 110 may display (e.g., a flash of a few milliseconds (ms)) a frame including only a red color component as illustrated by example 202, a frame including only a green color component as illustrated by example 204, and a frame including only a blue color component as illustrated by example 206. In one embodiment, in lieu of the display 110 displaying the sequence of frames to the user, one or more light sources (e.g., a number of (R)ed, (G)reen, (B)lue LEDs) of XR display device 100 may project a sequence of optical pulses onto the user. For example, the sequence of optical pulses (e.g., illustrated by red light 208A, green light 208B, and blue light 208C) may include a first optical pulse including only the red color component, a second optical pulse including only the green color component, and a third optical pulse frame including only the blue color component.

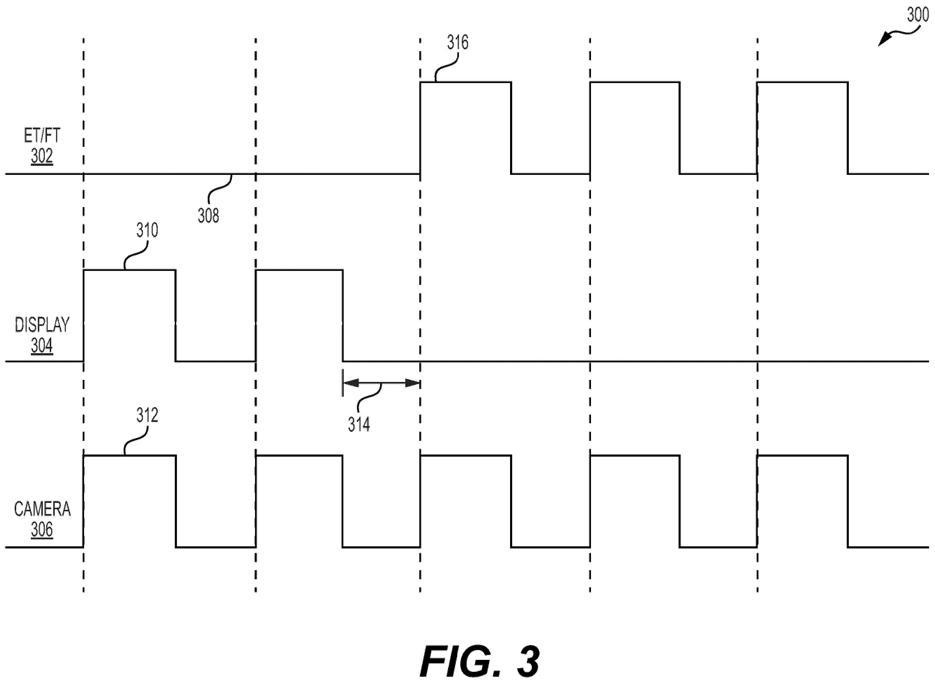

In certain embodiments, while displaying the sequence of red, green, and blue frames to the user, the one or monochromatic cameras 126B of the XR display device 100 may capture a number of images of the user. Specifically, the one or monochromatic cameras 126B may capture reflections 210A, 210B, and 210C of the red light 208A, the green light 208B, and the blue light 208C. For example, in some embodiments, as will be further appreciated with respect to FIG. 3, the one or monochromatic cameras 126B may capture a first image of the user while the display 110 is displaying the first frame including the red color component, capture a second image of the user while the display 110 is displaying the second frame including the green color component, and capture a third image of the user while the display 110 displaying the third frame including the blue color component in time-synchronized sequence.

In certain embodiments, the XR display device 100 may then determine, for each of the captured images of the user, a visible wavelength for each of the red color component, the green color component, and the blue color component. For example, the XR display device 100 may detect or calculate the visible wavelength for each of the red color component, the green color component, and the blue color component based on the reflections 210A, 210B, and 210C of the red light 208A, the green light 208B, and the blue light 208C captured by the one or monochromatic cameras 126B. In certain embodiments, the XR display device 100 may utilize the detected or calculated visible wavelengths to determine a color for each of a number of characteristics of the user. For example, in some embodiments, the number of characteristics of the user may include one or more of an eye color of the user, a head hair color of the user, a skin complexion of the user, a facial hair color of the user, or a cheek complexion of the user in accordance with the presently disclosed techniques.

In certain embodiments, the XR display device 100 may then generate a realistic avatar of the user based on the visible wavelength for each of the red color component, the green color component, and the blue color component. For example, in some embodiments, the XR display device 100 may generate the realistic avatar of the user by combining the images of the captured reflections 210A, 210B, and 210C of the red light 208A, the green light 208B, and the blue light 208C (e.g., as depicted by RGB image 212). In another embodiment, based on the visible wavelength for each of the red color component, the green color component, and the blue color component, the XR display device 100 may also determine a heart rate of the user. In another embodiment, based on the visible wavelength for each of the red color component, the XR display device 100 may also determine a blood oxygen level of the user.

**FIG.** 3 illustrates a timing diagram 300 that shows the timing signals 302, 304, and 306 of an eye-tracking and face-tracking functionality operation, a display operation, and a camera operation (e.g., one or more monochromatic cameras), respectively, in accordance with the present embodiments. In certain embodiments, the timing signal 302 (e.g., eye-tracking and face-tracking functionality operation), the timing signal 304 (e.g., display operation), and the timing signal 306 (e.g., camera operation) may each include rising edges, which may indicate, for example, when the eye-tracking and face-tracking functionality operates to track the user's eye 120 or the user's face 124A, 124B, when the display 110 operates to display (e.g., a flash of a few ms) the sequence of a red frame, a green frame, and a blue frame, and when the one or more monochromatic cameras 126B are to operate to capture images of the user's eye 120 and the user's face 124A, 124B. For example, as depicted, in some embodiments, the timing signal 304 (e.g., display operation) and the timing signal 306 (e.g., camera operation) may be synchronized.

For example, in accordance with the present embodiments, the timing signal 302 (e.g., eye-tracking and face-tracking functionality operation) may begin logically low at signal portion 308, such that the eye-tracking and face-tracking functionality of the XR display device 100 may not operate while the functionality to generate a realistic avatar of a user utilizing detected or calculated visible wavelengths is operating. Specifically, as further depicted, the timing signal 304 (e.g., display operation) and the timing signal 306 (e.g., camera operation) may be logically high at signal portions 310 and 312, respectively, and logically low at substantially the same time, such that the display 110 and the one or more monochromatic cameras 126B, for example, operates in accordance with the following sequence: the display 110 displays a red frame (e.g., an all red screen or a bright flash of red light) to the user, and while the display 110 is displaying the red frame to the user, the one or monochromatic cameras 126B captures a first image of the user; the display 110 then displays a green frame (e.g., an all green screen or a bright flash of green light) to the user, and while the display 110 is displaying the green frame to the user, the one or monochromatic cameras 126B captures a second image of the user; and finally the display 110 displays a blue frame (e.g., an all blue screen or a bright flash of blue light) to the user, and while the display 110 is displaying the blue frame to the user, the one or monochromatic cameras 126B captures a third image of the user.

In certain embodiments, upon the timing signal 304 (e.g., display operation) and the timing signal 306 (e.g., camera operation) turning logically low at signal portion 314 (e.g., after the display 110 displays the blue frame to the user and the one or monochromatic cameras 126B captures third image of the user), the timing signal 302 (e.g., eye-tracking and face-tracking functionality operation) may turn logically high at signal portion 316 and then operate in synchronization with the timing signal 306 (e.g., camera operation) to perform, for example, eye-tracking and face-tracking of the user. In this way, by offsetting the operations of the eye-tracking and face-tracking functionality and the functionality to generate a realistic avatar of a user utilizing detected or calculated visible wavelengths as described herein, the sequence of displaying a red frame, a green frame, and blue frame by the display 110 may not adversely impact the eye-tracking and face-tracking functionality of the XR display device 100.

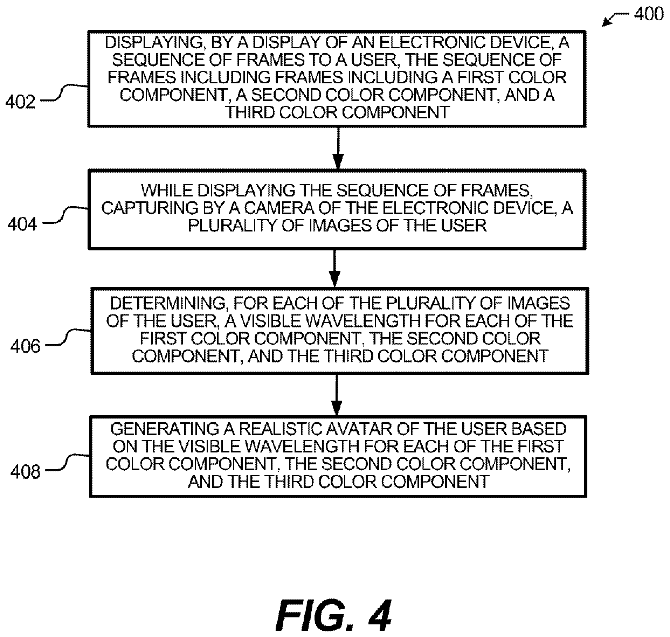

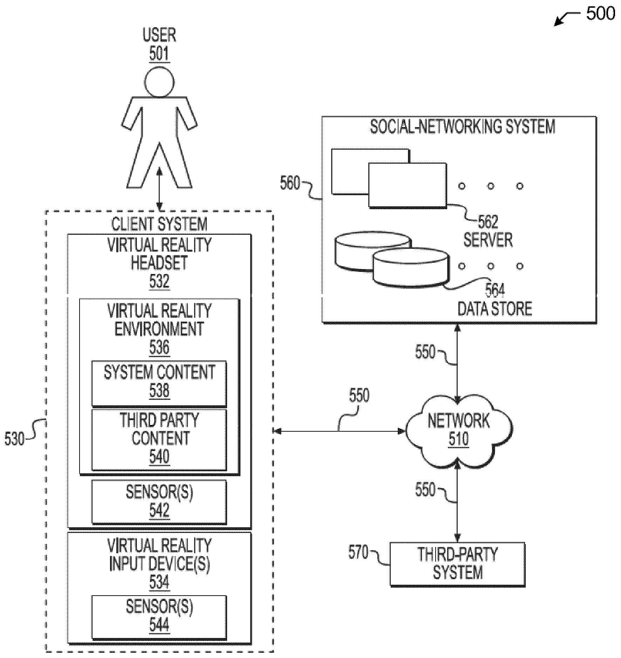

**FIG.** 4 illustrates is a flow diagram of a method 400 for generating a realistic avatar of a user, in accordance with the presently disclosed embodiments. The method 400 may be performed utilizing one or more processors that may include hardware (e.g., a general purpose processor, a graphic processing units (GPU), an application-specific integrated circuit (ASIC), a system-on-chip (SoC), a microcontroller, a field-programmable gate array (FPGA), or any other processing device(s) that may be suitable for processing image data), software (e.g., instructions running/executing on one or more processors), firmware (e.g., microcode), or any combination thereof.

The method 400 may begin at block 402 with one or more processors accessing displaying, by at least one display of an electronic device, a sequence of frames to a user of the electronic device. For example, the sequence of frames may include a first frame including a first color component, a second frame including a second color component, and third frame including a third color component. The method 400 may continue at block 404 with one or more processors while displaying the sequence of frames to the user, capturing, by one or more cameras of the electronic device, a plurality of images of the user. The method 400 may continue at block 406 with one or more processors determining, for each of the plurality of images of the user, a visible wavelength for each of the first color component, the second color component, and the third color component. The method 400 may conclude at block 408 with one or more processors generating a realistic avatar of the user based on the visible wavelength for each of the first color component, the second color component, and the third color component.

FIG. 5 illustrates an example network environment 500 associated with a virtual reality system. Network environment 500 includes a user 501 interacting with a client system 530, a social-networking system 560, and a third-party system 570 connected to each other by a network 510. Although FIG. 5 illustrates a particular arrangement of a user 501, a client system 530, a social-networking system 560, a third-party system 570, and a network 510, this disclosure contemplates any suitable arrangement of a user 501, a client system 530, a social-networking system 560, a third-party system 570, and a network 510. As an example, and not by way of limitation, two or more of users 501, a client system 530, a social-networking system 560, and a third-party system 570 may be connected to each other directly, bypassing a network 510. As another example, two or more of client systems 530, a social-networking system 560, and a third-party system 570 may be physically or logically co-located with each other in whole or in part. Moreover, although FIG. 5 illustrates a particular number of users 501, client systems 530, social-networking systems 560, third-party systems 570, and networks 510, this disclosure contemplates any suitable number of client systems 530, social-networking systems 560, third-party systems 570, and networks 510. As an example, and not by way of limitation, network environment 500 may include multiple users 501, client systems 530, social-networking systems 560, third-party systems 570, and networks 510.

This disclosure contemplates any suitable network 510. As an example, and not by way of limitation, one or more portions of a network 510 may include an ad hoc network, an intranet, an extranet, a virtual private network (VPN), a local area network (LAN), a wireless LAN (WLAN), a wide area network (WAN), a wireless WAN (WWAN), a metropolitan area network (MAN), a portion of the Internet, a portion of the Public Switched Telephone Network (PSTN), a cellular telephone network, or a combination of two or more of these. A network 510 may include one or more networks 510. Links 550 may connect a client system 530, a social-networking system 560, and a third-party system 570 to a communication network 510 or to each other. This disclosure contemplates any suitable links 550. In certain embodiments, one or more links 550 include one or more wireline (such as for example Digital Subscriber Line (DSL) or Data Over Cable Service Interface Specification (DOCSIS)), wireless (such as for example Wi-Fi or Worldwide Interoperability for Microwave Access (WiMAX)), or optical (such as for example Synchronous Optical Network (SONET) or Synchronous Digital Hierarchy (SDH)) links. In certain embodiments, one or more links 550 each include an ad hoc network, an intranet, an extranet, a VPN, a LAN, a WLAN, a WAN, a WWAN, a MAN, a portion of the Internet, a portion of the PSTN, a cellular technology-based network, a satellite communications technology-based network, another link 550, or a combination of two or more such links 550. Links 550 need not necessarily be the same throughout a network environment 500. One or more first links 550 may differ in one or more respects from one or more second links 550.

In certain embodiments, a client system 530 may be an electronic device including hardware, software, or embedded logic components or a combination of two or more such components and capable of carrying out the appropriate functionalities implemented or supported by a client system 530. As an example, and not by way of limitation, a client system 530 may include a computer system such as a desktop computer, notebook or laptop computer, netbook, a tablet computer, e-book reader, GPS device, camera, a digital assistant (PDA), handheld electronic device, cellular telephone, smartphone, virtual reality headset and controllers, other suitable electronic device, or any suitable combination thereof. This disclosure contemplates any suitable client systems 530. A client system 530 may enable a network user at a client system 530 to access a network 510. A client system 530 may enable its user to communicate with other users at other client systems 530. A client system 530 may generate a virtual reality environment for a user to interact with content.

In certain embodiments, a client system 530 may include a virtual reality (or augmented reality) headset 532, and virtual reality input device(s) 534, such as a virtual reality controller. A user at a client system 530 may wear the virtual reality headset 532 and use the virtual reality input device(s) to interact with a virtual reality environment 536 generated by the virtual reality headset 532. Although not shown, a client system 530 may also include a separate processing computer and/or any other component of a virtual reality system. A virtual reality headset 532 may generate a virtual reality environment 536, which may include system content 538 (including but not limited to the operating system), such as software or firmware updates and also include third-party content 540, such as content from applications or dynamically downloaded from the Internet (e.g., web page content). A virtual reality headset 532 may include sensor(s) 542, such as accelerometers, gyroscopes, magnetometers to generate sensor data that tracks the location of the headset device 532. The headset 532 may also include eye trackers for tracking the position of the user's eyes or their viewing directions. The client system may use data from the sensor(s) 542 to determine velocity, orientation, and gravitation forces with respect to the headset.

Virtual reality input device(s) 534 may include sensor(s) 544, such as accelerometers, gyroscopes, magnetometers, and touch sensors to generate sensor data that tracks the location of the input device 534 and the positions of the user's fingers. The client system 530 may make use of outside-in tracking, in which a tracking camera (not shown) is placed external to the virtual reality headset 532 and within the line of sight of the virtual reality headset 532. In outside-in tracking, the tracking camera may track the location of the virtual reality headset 532 (e.g., by tracking one or more infrared LED markers on the virtual reality headset 532). Alternatively, or additionally, the client system 530 may make use of inside-out tracking, in which a tracking camera (not shown) may be placed on or within the virtual reality headset 532 itself. In inside-out tracking, the tracking camera may capture images around it in the real world and may use the changing perspectives of the real world to determine its own position in space.

Third-party content 540 may include a web browser, such as MICROSOFT INTERNET EXPLORER, GOOGLE CHROME or MOZILLA FIREFOX, and may have one or more add-ons, plug-ins, or other extensions, such as TOOLBAR or YAHOO TOOLBAR. A user at a client system 530 may enter a Uniform Resource Locator (URL) or other address directing a web browser to a particular server (such as server 562, or a server associated with a third-party system 570), and the web browser may generate a Hyper Text Transfer Protocol (HTTP) request and communicate the HTTP request to server. The server may accept the HTTP request and communicate to a client system 530 one or more Hyper Text Markup Language (HTML) files responsive to the HTTP request. The client system 530 may render a web interface (e.g., a webpage) based on the HTML files from the server for presentation to the user. This disclosure contemplates any suitable source files. As an example, and not by way of limitation, a web interface may be rendered from HTML files, Extensible Hyper Text Markup Language (XHTML) files, or Extensible Markup Language (XML) files, according to particular needs. Such interfaces may also execute scripts such as, for example and without limitation, those written in JAVASCRIPT, JAVA, MICROSOFT SILVERLIGHT, combinations of markup language and scripts such as AJAX (Asynchronous JAVASCRIPT and XML), and the like. Herein, reference to a web interface encompasses one or more corresponding source files (which a browser may use to render the web interface) and vice versa, where appropriate.

**In** certain embodiments, the social-networking system 560 may be a network-addressable computing system that may host an online social network. The social-networking system 560 may generate, store, receive, and send social-networking data, such as, for example, user-profile data, concept-profile data, social-graph information, or other suitable data related to the online social network. The social-networking system 560 may be accessed by the other components of network environment 500 either directly or via a network 510. As an example, and not by way of limitation, a client system 530 may access the social-networking system 560 using a web browser of a third-party content 540, or a native application associated with the social-networking system 560 (e.g., a mobile social-networking application, a messaging application, another suitable application, or any combination thereof) either directly or via a network 510. **In** certain embodiments, the social-networking system 560 may include one or more servers 562. Each server 562 may be a unitary server or a distributed server spanning multiple computers or multiple datacenters. Servers 562 may be of various types, such as, for example and without limitation, web server, news server, mail server, message server, advertising server, file server, application server, exchange server, database server, proxy server, another server suitable for performing functions or processes described herein, or any combination thereof.

**In** certain embodiments, each server 562 may include hardware, software, or embedded logic components or a combination of two or more such components for carrying out the appropriate functionalities implemented or supported by server 562. **In** certain embodiments, the social-networking system 560 may include one or more data stores 564. Data stores 564 may be used to store various types of information. **In** certain embodiments, the information stored in data stores 564 may be organized according to specific data structures. **In** certain embodiments, each data store 564 may be a relational, columnar, correlation, or other suitable database. Although this disclosure describes or illustrates particular types of databases, this disclosure contemplates any suitable types of databases. Certain embodiments may provide interfaces that enable a client system 530, a social-networking system 560, or a third-party system 570 to manage, retrieve, modify, add, or delete, the information stored in data store 564.

**In** certain embodiments, the social-networking system 560 may store one or more social graphs in one or more data stores 564. **In** certain embodiments, a social graph may include multiple nodes-which may include multiple user nodes (each corresponding to a particular user) or multiple concept nodes (each corresponding to a particular concept)-and multiple edges connecting the nodes. The social-networking system 560 may provide users of the online social network the ability to communicate and interact with other users. In certain embodiments, users may join the online social network via the social-networking system 560 and then add connections (e.g., relationships) to a number of other users of the social-networking system 560 whom they want to be connected to. Herein, the term "friend" may refer to any other user of the social-networking system 560 with whom a user has formed a connection, association, or relationship via the social-networking system 560.

In certain embodiments, the social-networking system 560 may provide users with the ability to take actions on various types of items or objects, supported by the social-networking system 560. As an example, and not by way of limitation, the items and objects may include groups or social networks to which users of the social-networking system 560 may belong, events or calendar entries in which a user might be interested, computer-based applications that a user may use, transactions that allow users to buy or sell items via the service, interactions with advertisements that a user may perform, or other suitable items or objects. A user may interact with anything that is capable of being represented in the social-networking system 560 or by an external system of a third-party system 570, which is separate from the social-networking system 560 and coupled to the social-networking system 560 via a network 510.

In certain embodiments, the social-networking system 560 may be capable of linking a variety of entities. As an example, and not by way of limitation, the social-networking system 560 may enable users to interact with each other as well as receive content from third-party systems 570 or other entities, or to allow users to interact with these entities through an application programming interfaces (API) or other communication channels. In certain embodiments, a third-party system 570 may include one or more types of servers, one or more data stores, one or more interfaces, including but not limited to APIs, one or more web services, one or more content sources, one or more networks, or any other suitable components, e.g., that servers may communicate with. A third-party system 570 may be operated by a different entity from an entity operating the social-networking system 560. In certain embodiments, however, the social-networking system 560 and third-party systems 570 may operate in conjunction with each other to provide social-networking services to users of the social-networking system 560 or third-party systems 570. In this sense, the social-networking system 560 may provide a platform, or backbone, which other systems, such as third-party systems 570, may use to provide social-networking services and functionality to users across the Internet.

In certain embodiments, a third-party system 570 may include a third-party content object provider. A third-party content object provider may include one or more sources of content objects, which may be communicated to a client system 530. As an example, and not by way of limitation, content objects may include information regarding things or activities of interest to the user, such as, for example, movie show times, movie reviews, restaurant reviews, restaurant menus, product information and reviews, or other suitable information. As another example and not by way of limitation, content objects may include incentive content objects, such as coupons, discount tickets, gift certificates, or other suitable incentive objects.

In certain embodiments, the social-networking system 560 also includes user-generated content objects, which may enhance a user's interactions with the social-networking system 560. User-generated content may include anything a user may add, upload, send, or "post" to the social-networking system 560. As an example, and not by way of limitation, a user communicates posts to the social-networking system 560 from a client system 530. Posts may include data such as status updates or other textual data, location information, photos, videos, links, music or other similar data or media. Content may also be added to the social-networking system 560 by a third-party through a "communication channel," such as a news feed or stream. In certain embodiments, the social-networking system 560 may include a variety of servers, sub-systems, programs, modules, logs, and data stores. In certain embodiments, the social-networking system 560 may include one or more of the following: a web server, action logger, API-request server, relevance-and-ranking engine, content-object classifier, notification controller, action log, third-party-content-object-exposure log, inference module, authorization/privacy server, search module, advertisement-targeting module, user-interface module, user-profile store, connection store, third-party content store, or location store. The social-networking system 560 may also include suitable components such as network interfaces, security mechanisms, load balancers, failover servers, management-and-network-operations consoles, other suitable components, or any suitable combination thereof.

**In** certain embodiments, the social-networking system 560 may include one or more user-profile stores for storing user profiles. A user profile may include, for example, biographic information, demographic information, behavioral information, social information, or other types of descriptive information, such as work experience, educational history, hobbies or preferences, interests, affinities, or location. Interest information may include interests related to one or more categories. Categories may be general or specific. As an example, and not by way of limitation, if a user "likes" an article about a brand of shoes the category may be the brand, or the general category of "shoes" or "clothing." A connection store may be used for storing connection information about users. The connection information may indicate users who have similar or common work experience, group memberships, hobbies, educational history, or are in any way related or share common attributes. The connection information may also include user-defined connections between different users and content (both internal and external). A web server may be used for linking the social-networking system 560 to one or more client systems 530 or one or more third-party systems 570 via a network 510. The web server may include a mail server or other messaging functionality for receiving and routing messages between the social-networking system 560 and one or more client systems 530. An API-request server may allow a third-party system 570 to access information from the social-networking system 560 by calling one or more APIs. An action logger may be used to receive communications from a web server about a user's actions on or off the social-networking system 560.

**In** conjunction with the action log, a third-party-content-object log may be maintained of user exposures to third-party-content objects. A notification controller may provide information regarding content objects to a client system 530. Information may be pushed to a client system 530 as notifications, or information may be pulled from a client system 530 responsive to a request received from a client system 530. Authorization servers may be used to enforce one or more privacy settings of the users of the social-networking system 560. A privacy setting of a user may determine how particular information associated with a user may be shared. The authorization server may allow users to opt in to or opt out of having their actions logged by the social-networking system 560 or shared with other systems (e.g., a third-party system 570), such as, for example, by setting appropriate privacy settings. Third-party-content-object stores may be used to store content objects received from third parties, such as a third-party system 570. Location stores may be used for storing location information received from client systems 530 associated with users. Advertisement-pricing modules may combine social information, the current time, location information, or other suitable information to provide relevant advertisements, in the form of notifications, to a user.

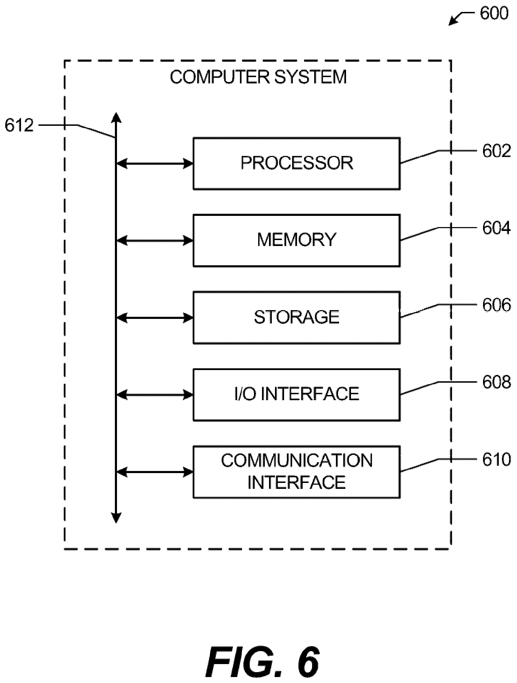

**FIG. 6** illustrates an example computer system 600 that may be useful in performing one or more of the foregoing techniques as presently disclosed herein. **In** certain embodiments, one or more computer systems 600 perform one or more steps of one or more methods described or illustrated herein. **In** certain embodiments, one or more computer systems 600 provide functionality described or illustrated herein. **In** certain embodiments, software running on one or more computer systems 600 performs one or more steps of one or more methods described or illustrated herein or provides functionality described or illustrated herein. Certain embodiments include one or more portions of one or more computer systems 600. Herein, reference to a computer system may encompass a computing device, and vice versa, where appropriate. Moreover, reference to a computer system may encompass one or more computer systems, where appropriate.

This disclosure contemplates any suitable number of computer systems 600. This disclosure contemplates computer system 600 taking any suitable physical form. As example and not by way of limitation, computer system 600 may be an embedded computer system, a system-on-chip (SOC), a single-board computer system (SBC) (such as, for example, a computer-on-module (COM) or system-on-module (SOM)), a desktop computer system, a laptop or notebook computer system, an interactive kiosk, a mainframe, a mesh of computer systems, a mobile telephone, a digital assistant (PDA), a server, a tablet computer system, an augmented/virtual reality device, or a combination of two or more of these. Where appropriate, computer system 600 may include one or more computer systems 600; be unitary or distributed; span multiple locations; span multiple machines; span multiple data centers; or reside in a cloud, which may include one or more cloud components in one or more networks. Where appropriate, one or more computer systems 600 may perform without substantial spatial or temporal limitation one or more steps of one or more methods described or illustrated herein.

As an example, and not by way of limitation, one or more computer systems 600 may perform in real time or in batch mode one or more steps of one or more methods described or illustrated herein. One or more computer systems 600 may perform at different times or at different locations one or more steps of one or more methods described or illustrated herein, where appropriate. In certain embodiments, computer system 600 includes a processor 602, memory 604, storage 606, an input/output (I/O) interface 608, a communication interface 610, and a bus 612. Although this disclosure describes and illustrates a particular computer system having a particular number of particular components in a particular arrangement, this disclosure contemplates any suitable computer system having any suitable number of any suitable components in any suitable arrangement.

**In** certain embodiments, processor 602 includes hardware for executing instructions, such as those making up a computer program. As an example, and not by way of limitation, to execute instructions, processor 602 may retrieve (or fetch) the instructions from an internal register, an internal cache, memory 604, or storage 606; decode and execute them; and then write one or more results to an internal register, an internal cache, memory 604, or storage 606. In certain embodiments, processor 602 may include one or more internal caches for data, instructions, or addresses. This disclosure contemplates processor 602 including any suitable number of any suitable internal caches, where appropriate. As an example, and not by way of limitation, processor 602 may include one or more instruction caches, one or more data caches, and one or more translation lookaside buffers (TLBs). Instructions in the instruction caches may be copies of instructions in memory 604 or storage 606, and the instruction caches may speed up retrieval of those instructions by processor 602.

Data in the data caches may be copies of data in memory 604 or storage 606 for instructions executing at processor 602 to operate on; the results of previous instructions executed at processor 602 for access by subsequent instructions executing at processor 602 or for writing to memory 604 or storage 606; or other suitable data. The data caches may speed up read or write operations by processor 602. The TLBs may speed up virtual-address translation for processor 602. In certain embodiments, processor 602 may include one or more internal registers for data, instructions, or addresses. This disclosure contemplates processor 602 including any suitable number of any suitable internal registers, where appropriate. Where appropriate, processor 602 may include one or more arithmetic logic units (ALUs); be a multi-core processor; or include one or more processors 602. Although this disclosure describes and illustrates a particular processor, this disclosure contemplates any suitable processor.

In certain embodiments, memory 604 includes main memory for storing instructions for processor 602 to execute or data for processor 602 to operate on. As an example, and not by way of limitation, computer system 600 may load instructions from storage 606 or another source (such as, for example, another computer system 600) to memory 604. Processor 602 may then load the instructions from memory 604 to an internal register or internal cache. To execute the instructions, processor 602 may retrieve the instructions from the internal register or internal cache and decode them. During or after execution of the instructions, processor 602 may write one or more results (which may be intermediate or final results) to the internal register or internal cache. Processor 602 may then write one or more of those results to memory 604. In certain embodiments, processor 602 executes only instructions in one or more internal registers or internal caches or in memory 604 (as opposed to storage 606 or elsewhere) and operates only on data in one or more internal registers or internal caches or in memory 604 (as opposed to storage 606 or elsewhere).

One or more memory buses (which may each include an address bus and a data bus) may couple processor 602 to memory 604. Bus 612 may include one or more memory buses, as described below. In certain embodiments, one or more memory management units (MMUs) reside between processor 602 and memory 604 and facilitate accesses to memory 604 requested by processor 602. In certain embodiments, memory 604 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 604 may include one or more memories 604, where appropriate. Although this disclosure describes and illustrates particular memory, this disclosure contemplates any suitable memory.

In certain embodiments, storage 606 includes mass storage for data or instructions. As an example, and not by way of limitation, storage 606 may include a hard disk drive (HDD), a floppy disk drive, flash memory, an optical disc, a magneto-optical disc, magnetic tape, or a Universal Serial Bus (USB) drive or a combination of two or more of these. Storage 606 may include removable or non-removable (or fixed) media, where appropriate. Storage 606 may be internal or external to computer system 600, where appropriate. In certain embodiments, storage 606 is non-volatile, solid-state memory. In certain embodiments, storage 606 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass storage 606 taking any suitable physical form. Storage 606 may include one or more storage control units facilitating communication between processor 602 and storage 606, where appropriate. Where appropriate, storage 606 may include one or more storages 606. Although this disclosure describes and illustrates particular storage, this disclosure contemplates any suitable storage.

In certain embodiments, I/O interface 608 includes hardware, software, or both, providing one or more interfaces for communication between computer system 600 and one or more I/O devices. Computer system 600 may include one or more of these I/O devices, where appropriate. One or more of these I/O devices may enable communication between a user and computer system 600. As an example, and not by way of limitation, an I/O device may include a keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 608 for them. Where appropriate, I/O interface 608 may include one or more device or software drivers enabling processor 602 to drive one or more of these I/O devices. I/O interface 608 may include one or more I/O interfaces 608, where appropriate. Although this disclosure describes and illustrates a particular I/O interface, this disclosure contemplates any suitable I/O interface.

In certain embodiments, communication interface 610 includes hardware, software, or both providing one or more interfaces for communication (such as, for example, packet-based communication) between computer system 600 and one or more other computer systems 600 or one or more networks. As an example, and not by way of limitation, communication interface 610 may include a network interface controller (NIC) or network adapter for communicating with an Ethernet or other wire-based network or a wireless NIC (WNIC) or wireless adapter for communicating with a wireless network, such as a Wi-Fi network. This disclosure contemplates any suitable network and any suitable communication interface 610 for it.

As an example, and not by way of limitation, computer system 600 may communicate with an ad hoc network, an area network (PAN), a local area network (LAN), a wide area network (WAN), a metropolitan area network (MAN), or one or more portions of the Internet or a combination of two or more of these. One or more portions of one or more of these networks may be wired or wireless. As an example, computer system 600 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. Computer system 600 may include any suitable communication interface 610 for any of these networks, where appropriate. Communication interface 610 may include one or more communication interfaces 610, where appropriate. Although this disclosure describes and illustrates a particular communication interface, this disclosure contemplates any suitable communication interface.

In certain embodiments, bus 612 includes hardware, software, or both coupling components of computer system 600 to each other. As an example, and not by way of limitation, bus 612 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 612 may include one or more buses 612, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

The scope of this disclosure encompasses all changes, substitutions, variations, alterations, and modifications to the example embodiments described or illustrated herein that a user having ordinary skill in the art would comprehend. The scope of this disclosure is not limited to the example embodiments described or illustrated herein. Moreover, although this disclosure describes and illustrates respective embodiments herein as including particular components, elements, feature, functions, operations, or steps, any of these embodiments may include any combination or permutation of any of the components, elements, features, functions, operations, or steps described or illustrated anywhere herein that a user having ordinary skill in the art would comprehend. Additionally, although this disclosure describes or illustrates certain embodiments as providing particular advantages, certain embodiments may provide none, some, or all of these advantages.

## Claims

1. A method for generating a realistic avatar of a user, comprising, by an electronic device (100):
displaying, by at least one display (110) of the electronic device (100), a sequence of frames to a user of the electronic device (100), wherein the sequence of frames comprises a first frame including a first color component, a second frame including a second color component, and a third frame including a third color component;
while displaying the sequence of frames to the user, capturing, by one or more cameras (126A, 126B, 126C) of the electronic device (100), a plurality of images of the user based on reflections (210A, 210B, 210C) from the sequence of frames displayed to the user; and
generating a realistic avatar of the user by combining the images of the captured reflections (210A, 210B, 210C).

2. The method of Claim 1, wherein:
the first color component comprises a red color component;
the second color component comprises a green color component; and
the third color component comprises a blue color component;
and for example, wherein:
the first frame includes only the red color component;
the second frame includes only the green color component; and
third frame includes only the blue color component.

3. The method of Claim 1 or claim 2, wherein determining, for each of the plurality of images of the user, the visible wavelengths comprises determining a color for each of a plurality of characteristics of the user.

4. The method of Claim 3, wherein the plurality of characteristics of the user comprises one or more of an eye color of the user, a head hair color of the user, a skin complexion of the user, a facial hair color of the user, or a cheek complexion of the user.

5. The method of any preceding Claim, wherein generating the realistic avatar of the user comprises combining the plurality of images of the user based on the visible wavelength for each of the first color component, the second color component, and the third color component.

6. The method of any preceding Claim, wherein capturing, by the one or more cameras (126A, 126B, 126C), the plurality of images of the user further comprises:
capturing a first image of the plurality of images of the user while displaying the first frame including the first color component;
capturing a second image of the plurality of images of the user while displaying the second frame including the second color component; and
capturing a third image of the plurality of images of the user while displaying the third frame including the third color component.

7. The method of any preceding Claim, further comprising determining a heart rate of the user based on the visible wavelength for each of the first color component, the second color component, and the third color component.

8. The method of any preceding Claim, further comprising determining a blood oxygen level of the user based on the visible wavelength for each of the first color component, the second color component, and the third color component.

9. An electronic device (100), comprising:
at least one display (110);
one or more cameras (126A, 126B, 126C);
one or more non-transitory computer-readable storage media including instructions; and
one or more processors (602) coupled to the storage media and the one or more cameras (126A, 126B, 126C), the one or more processors (602) configured to execute the instructions to:
display, by the at least one display (110), a sequence of frames to a user of the electronic device (100), wherein the sequence of frames comprises a first frame including a first color component, a second frame including a second color component, and a third frame including a third color component;
while displaying the sequence of images to the user, capture, by the one or more cameras (126A, 126B, 126C), a plurality of images of the user based on reflections (210A, 210B, 210C) from the sequence of frames displayed to the user; and
generate a realistic avatar of the user by combining the images of the captured reflections (210A, 210B, 210C).

10. The electronic device (100) of Claim 9, wherein:
the first color component comprises a red color component;
the second color component comprises a green color component; and
the third color component comprises a blue color component;
and for example, wherein:
the first frame includes only the red color component;
the second frame includes only the green color component; and
third frame includes only the blue color component.

11. The electronic device of Claim 9 or 10, wherein the instructions to determine, for each of the plurality of images of the user, the visible wavelengths further comprise instructions to determine a color for each of a plurality of characteristics of the user.

12. The electronic device (100) of Claim 11, wherein the plurality of characteristics of the user comprises one or more of an eye color of the user, a head hair color of the user, a skin complexion of the user, a facial hair color of the user, or a cheek complexion of the user.

13. The electronic device (100) of one of Claims 9 to 12, wherein the instructions further comprise instructions to cause the device to perform the method of any one of claims 4 to 8.

14. A non-transitory computer-readable medium comprising instructions that, when executed by one or more processors (602) of an electronic device (100), cause the electronic device (100) to:
display, by at least one display (110) of the electronic device (100), a sequence of frames to a user of the electronic device (100), wherein the sequence of frames comprises a first frame including a first color component, a second frame including a second color component, and a third frame including a third color component;
while displaying the sequence of images to the user, capture, by one or more cameras (126A, 126B, 126C) of the electronic device (100), a plurality of images of the user based on reflections (210A, 210B, 210C) from the sequence of frames displayed to the user; and
generate a realistic avatar of the user by combining the images of the captured reflections (210A, 210B, 210C).

15. The non-transitory computer-readable medium of claim 14 comprising instructions that, when executed by one or more processors (602) of an electronic device (100), cause the electronic device (100) to perform the method of any one of claims 2 to 8.

## Patentansprüche

1. Ein Verfahren zum Erzeugen eines realistischen Avatars eines Benutzers, beinhaltend, durch eine elektronische Vorrichtung (100):
Anzeigen, durch mindestens eine Anzeige (110) der elektronischen Vorrichtung (100), einer Sequenz von Einzelbildern für einen Benutzer der elektronischen Vorrichtung (100), wobei die Sequenz von Einzelbildern ein erstes Einzelbild, das eine erste Farbkomponente umfasst, ein zweites Einzelbild, das eine zweite Farbkomponente umfasst, und ein drittes Einzelbild, das eine dritte Farbkomponente umfasst, beinhaltet;
während des Anzeigens der Sequenz von Einzelbildern für den Benutzer, Erfassen, durch eine oder mehrere Kameras (126A, 126B, 126C) der elektronischen Vorrichtung (100), einer Vielzahl von Bildern des Benutzers auf der Basis von Reflexionen (210A, 210B, 210C) von der Sequenz von Einzelbildern, die dem Benutzer angezeigt werden; und
Erzeugen eines realistischen Avatars des Benutzers durch Kombinieren der Bilder der erfassten Reflexionen (210A, 210B, 210C).

2. Verfahren gemäß Anspruch 1, wobei:
die erste Farbkomponente eine rote Farbkomponente beinhaltet;
die zweite Farbkomponente eine grüne Farbkomponente beinhaltet; und
die dritte Farbkomponente eine blaue Farbkomponente beinhaltet;
und wobei zum Beispiel:
das erste Einzelbild nur die rote Farbkomponente umfasst;
das zweite Einzelbild nur die grüne Farbkomponente umfasst; und
das dritte Einzelbild nur die blaue Farbkomponente umfasst.

3. Verfahren gemäß Anspruch 1 oder Anspruch 2, wobei ein Bestimmen, für jedes der Vielzahl von Bildern des Benutzers, der sichtbaren Wellenlängen das Bestimmen einer Farbe für jedes einer Vielzahl von Merkmalen des Benutzers beinhaltet.

4. Verfahren gemäß Anspruch 3, wobei die Vielzahl von Merkmalen des Benutzers eines oder mehrere von einer Augenfarbe des Benutzers, einer Kopfhaarfarbe des Benutzers, einem Hautton des Benutzers, einer Gesichtshaarfarbe des Benutzers oder einem Wangenton des Benutzers beinhaltet.

5. Verfahren gemäß einem der vorhergehenden Ansprüche, wobei das Erzeugen des realistischen Avatars des Benutzers das Kombinieren der Vielzahl von Bildern des Benutzers auf der Basis der sichtbaren Wellenlänge für jede von der ersten Farbkomponente, der zweiten Farbkomponente und der dritten Farbkomponente beinhaltet.

6. Verfahren gemäß einem der vorhergehenden Ansprüche, wobei das Erfassen, durch die eine oder die mehreren Kameras (126A, 126B, 126C), der Vielzahl von Bildern des Benutzers ferner Folgendes beinhaltet:
Erfassen eines ersten Bildes der Vielzahl von Bildern des Benutzers während des Anzeigens des ersten Einzelbildes, das die erste Farbkomponente umfasst;
Erfassen eines zweiten Bildes der Vielzahl von Bildern des Benutzers während des Anzeigens des zweiten Einzelbildes, das die zweite Farbkomponente umfasst; und
Erfassen eines dritten Bildes der Vielzahl von Bildern des Benutzers während des Anzeigens des dritten Einzelbildes, das die dritte Farbkomponente umfasst.

7. Verfahren gemäß einem der vorhergehenden Ansprüche, das ferner das Bestimmen einer Herzfrequenz des Benutzers auf der Basis der sichtbaren Wellenlänge für jede von der ersten Farbkomponente, der zweiten Farbkomponente und der dritten Farbkomponente beinhaltet.

8. Verfahren gemäß einem der vorhergehenden Ansprüche, das ferner das Bestimmen eines Blutsauerstoffspiegels des Benutzers auf der Basis der sichtbaren Wellenlänge für jede von der ersten Farbkomponente, der zweiten Farbkomponente und der dritten Farbkomponente beinhaltet.

9. Eine elektronische Vorrichtung (100), beinhaltend:
mindestens eine Anzeige (110);
eine oder mehrere Kameras (126A, 126B, 126C);
ein oder mehrere nichtflüchtige computerlesbare Speichermedien, die Anweisungen umfassen; und
einen oder mehrere Prozessoren (602), die mit den Speichermedien und der einen oder den mehreren Kameras (126A, 126B, 126C) gekoppelt sind, wobei der eine oder die mehreren Prozessoren (602) konfiguriert sind, um die Anweisungen für Folgendes auszuführen:
Anzeigen, durch die mindestens eine Anzeige (110), einer Sequenz von Einzelbildern für einen Benutzer der elektronischen Vorrichtung (100), wobei die Sequenz von Einzelbildern ein erstes Einzelbild, das eine erste Farbkomponente umfasst, ein zweites Einzelbild, das eine zweite Farbkomponente umfasst, und ein drittes Einzelbild, das eine dritte Farbkomponente umfasst, beinhaltet;
während des Anzeigens der Sequenz von Bildern für den Benutzer, Erfassen, durch die eine oder die mehreren Kameras (126A, 126B, 126C), einer Vielzahl von Bildern des Benutzers auf der Basis von Reflexionen (210A, 210B, 210C) von der Sequenz von Einzelbildern, die dem Benutzer angezeigt werden; und
Erzeugen eines realistischen Avatars des Benutzers durch Kombinieren der Bilder der erfassten Reflexionen (210A, 210B, 210C).

10. Elektronische Vorrichtung (100) gemäß Anspruch 9, wobei:
die erste Farbkomponente eine rote Farbkomponente beinhaltet;
die zweite Farbkomponente eine grüne Farbkomponente beinhaltet; und
die dritte Farbkomponente eine blaue Farbkomponente beinhaltet;
und wobei zum Beispiel:
das erste Einzelbild nur die rote Farbkomponente umfasst;
das zweite Einzelbild nur die grüne Farbkomponente umfasst; und
das dritte Einzelbild nur die blaue Farbkomponente umfasst.

11. Elektronische Vorrichtung gemäß Anspruch 9 oder 10, wobei die Anweisungen zum Bestimmen, für jedes der Vielzahl von Bildern des Benutzers, der sichtbaren Wellenlängen ferner Anweisungen zum Bestimmen einer Farbe für jedes einer Vielzahl von Merkmalen des Benutzers beinhalten.

12. Elektronische Vorrichtung (100) gemäß Anspruch 11, wobei die Vielzahl von Merkmalen des Benutzers eines oder mehrere von einer Augenfarbe des Benutzers, einer Kopfhaarfarbe des Benutzers, einem Hautton des Benutzers, einer Gesichtshaarfarbe des Benutzers oder einem Wangenton des Benutzers beinhaltet.

13. Elektronische Vorrichtung (100) gemäß einem der Ansprüche 9 bis 12, wobei die Anweisungen ferner Anweisungen beinhalten, um die Vorrichtung zu veranlassen, das Verfahren gemäß einem der Ansprüche 4 bis 8 durchzuführen.

14. Ein nichtflüchtiges computerlesbares Medium, das Anweisungen beinhaltet, die, wenn sie von einem oder mehreren Prozessoren (602) einer elektronischen Vorrichtung (100) ausgeführt werden, die elektronische Vorrichtung (100) zu Folgendem veranlassen:
Anzeigen, durch mindestens eine Anzeige (110) der elektronischen Vorrichtung (100), einer Sequenz von Einzelbildern für einen Benutzer der elektronischen Vorrichtung (100), wobei die Sequenz von Einzelbildern ein erstes Einzelbild, das eine erste Farbkomponente umfasst, ein zweites Einzelbild, das eine zweite Farbkomponente umfasst, und ein drittes Einzelbild, das eine dritte Farbkomponente umfasst, beinhaltet;
während des Anzeigens der Sequenz von Bildern für den Benutzer, Erfassen, durch eine oder mehrere Kameras (126A, 126B, 126C) der elektronischen Vorrichtung (100), einer Vielzahl von Bildern des Benutzers auf der Basis von Reflexionen (210A, 210B, 210C) von der Sequenz von Einzelbildern, die dem Benutzer angezeigt werden; und
Erzeugen eines realistischen Avatars des Benutzers durch Kombinieren der Bilder der erfassten Reflexionen (210A, 210B, 210C).

15. Nichtflüchtiges computerlesbares Medium gemäß Anspruch 14, das Anweisungen beinhaltet, die, wenn sie von einem oder mehreren Prozessoren (602) einer elektronischen Vorrichtung (100) ausgeführt werden, die elektronische Vorrichtung (100) veranlassen, das Verfahren gemäß einem der Ansprüche 2 bis 8 durchzuführen.

## Revendications

1. Un procédé de génération d'un avatar réaliste d'un utilisateur, comprenant, par un dispositif électronique (100) :
l'affichage, par au moins un affichage (110) du dispositif électronique (100), d'une séquence de trames à destination d'un utilisateur du dispositif électronique (100), la séquence de trames comprenant une première trame incluant une première composante de couleur, une deuxième trame incluant une deuxième composante de couleur, et une troisième trame incluant une troisième composante de couleur ;
pendant l'affichage de la séquence de trames à destination de l'utilisateur, la capture, par une ou plusieurs caméras (126A, 126B, 126C) du dispositif électronique (100), d'une pluralité d'images de l'utilisateur sur la base de réflexions (210A, 210B, 210C) depuis la séquence de trames affichées à destination de l'utilisateur ; et
la génération d'un avatar réaliste de l'utilisateur en combinant les images des réflexions capturées (210A, 210B, 210C).

2. Le procédé de la revendication 1, dans lequel :
la première composante de couleur comprend une composante de couleur rouge ;
la deuxième composante de couleur comprend une composante de couleur verte ; et
la troisième composante de couleur comprend une composante de couleur bleue ;
et par exemple, dans lequel :
la première trame inclut uniquement la composante de couleur rouge ;
la deuxième trame inclut uniquement la composante de couleur verte ; et
la troisième trame inclut uniquement la composante de couleur bleue.

3. Le procédé de la revendication 1 ou de la revendication 2, dans lequel la détermination, pour chacune de la pluralité d'images de l'utilisateur, des longueurs d'onde visibles comprend la détermination d'une couleur pour chacune d'une pluralité de caractéristiques de l'utilisateur.

4. Le procédé de la revendication 3, dans lequel la pluralité de caractéristiques de l'utilisateur comprend une ou plusieurs d'une couleur des yeux de l'utilisateur, d'une couleur des cheveux de la tête de l'utilisateur, d'un teint de la peau de l'utilisateur, d'une couleur des cheveux du visage de l'utilisateur, ou d'un teint des joues de l'utilisateur.

5. Le procédé de n'importe quelle revendication précédente, dans lequel la génération de l'avatar réaliste de l'utilisateur comprend la combinaison de la pluralité d'images de l'utilisateur sur la base de la longueur d'onde visible pour chacune de la première composante de couleur, de la deuxième composante de couleur, et de la troisième composante de couleur.

6. Le procédé de n'importe quelle revendication précédente, dans lequel la capture, par les une ou plusieurs caméras (126A, 126B, 126C), de la pluralité d'images de l'utilisateur comprend en outre :
la capture d'une première image de la pluralité d'images de l'utilisateur pendant l'affichage de la première trame incluant la première composante de couleur ;
la capture d'une deuxième image de la pluralité d'images de l'utilisateur pendant l'affichage de la deuxième trame incluant la deuxième composante de couleur ; et
la capture d'une troisième image de la pluralité d'images de l'utilisateur pendant l'affichage de la troisième trame incluant la troisième composante de couleur.

7. Le procédé de n'importe quelle revendication précédente, comprenant en outre la détermination d'une fréquence cardiaque de l'utilisateur sur la base de la longueur d'onde visible pour chacune de la première composante de couleur, de la deuxième composante de couleur, et de la troisième composante de couleur.

8. Le procédé de n'importe quelle revendication précédente, comprenant en outre la détermination d'un taux d'oxygène dans le sang de l'utilisateur sur la base de la longueur d'onde visible pour chacune de la première composante de couleur, de la deuxième composante de couleur, et de la troisième composante de couleur.

9. Un dispositif électronique (100), comprenant :
au moins un affichage (110) ;
une ou plusieurs caméras (126A, 126B, 126C) ;
un ou plusieurs supports de stockage lisibles par ordinateur non transitoires incluant des instructions ; et
un ou plusieurs processeurs (602) couplés aux supports de stockage et aux une ou
plusieurs caméras (126A, 126B, 126C), les uns ou plusieurs processeurs (602) étant configurés pour exécuter les instructions pour :
afficher, par l'au moins un affichage (110), une séquence de trames à destination d'un utilisateur du dispositif électronique (100), la séquence de trames comprenant une première trame incluant une première composante de couleur,
une deuxième trame incluant une deuxième composante de couleur, et une troisième trame incluant une troisième composante de couleur ;
pendant l'affichage de la séquence d'images à destination de l'utilisateur, capturer, par les une ou plusieurs caméras (126A, 126B, 126C), une pluralité d'images de l'utilisateur sur la base de réflexions (210A, 210B, 210C) depuis la séquence de trames affichées à destination de l'utilisateur ; et
générer un avatar réaliste de l'utilisateur en combinant les images des réflexions capturées (210A, 210B, 210C).

10. Le dispositif électronique (100) de la revendication 9, dans lequel :
la première composante de couleur comprend une composante de couleur rouge ;
la deuxième composante de couleur comprend une composante de couleur verte ; et
la troisième composante de couleur comprend une composante de couleur bleue ;
et par exemple, dans lequel :
la première trame inclut uniquement la composante de couleur rouge ;
la deuxième trame inclut uniquement la composante de couleur verte ; et
la troisième trame inclut uniquement la composante de couleur bleue.

11. Le dispositif électronique de la revendication 9 ou de la revendication 10, dans lequel les instructions pour déterminer, pour chacune de la pluralité d'images de l'utilisateur, les longueurs d'onde visibles comprennent en outre des instructions pour déterminer une couleur pour chacune d'une pluralité de caractéristiques de l'utilisateur.

12. Le dispositif électronique (100) de la revendication 11, dans lequel la pluralité de caractéristiques de l'utilisateur comprend une ou plusieurs d'une couleur des yeux de l'utilisateur, d'une couleur des cheveux de la tête de l'utilisateur, d'un teint de la peau de l'utilisateur, d'une couleur des cheveux du visage de l'utilisateur, ou d'un teint des joues de l'utilisateur.

13. Le dispositif électronique (100) de l'une des revendications 9 à 12, dans lequel les instructions comprennent en outre des instructions pour amener le dispositif à réaliser le procédé de n'importe laquelle des revendications 4 à 8.

14. Un support lisible par ordinateur non transitoire comprenant des instructions qui, lorsqu'elles sont exécutées par un ou plusieurs processeurs (602) d'un dispositif électronique (100), amènent le dispositif électronique (100) à :
afficher, par au moins un affichage (110) du dispositif électronique (100), une séquence de trames à destination d'un utilisateur du dispositif électronique (100), la séquence de trames comprenant une première trame incluant une première composante de couleur,
une deuxième trame incluant une deuxième composante de couleur, et une troisième trame incluant une troisième composante de couleur ;
pendant l'affichage de la séquence d'images à destination de l'utilisateur, capturer, par une ou plusieurs caméras (126A, 126B, 126C) du dispositif électronique (100), une pluralité d'images de l'utilisateur sur la base de réflexions (210A, 210B, 210C) depuis la séquence de trames affichées à destination de l'utilisateur ; et
générer un avatar réaliste de l'utilisateur en combinant les images des réflexions capturées (210A, 210B, 210C).

15. Le support lisible par ordinateur non transitoire de la revendication 14 comprenant des instructions qui, lorsqu'elles sont exécutées par un ou plusieurs processeurs (602) d'un dispositif électronique (100), amènent le dispositif électronique (100) à réaliser le procédé de n'importe laquelle des revendications 2 à 8.
